# EUROPÄISCHE PATENTANMELDUNG

(11) **EP 2 216 059 A1**
(43) Veröffentlichungstag der Anmeldung: **11.08.2010**
(21) Anmeldenummer: 09075053.0
(22) Anmeldetag: 04.02.2009
(51) Int. Cl.: A61M 1/10, A61M 25/01

(54) **Kathetereinrichtung mit einem Katheter und einer Betätigungseinrichtung**

(71) Anmelder: ECP Entwicklungsgesellschaft mbH, 12247 Berlin (DE)
(72) Erfinder: Scheckel, Mario, 13187 Berlin (DE)
(74) Vertreter: Pfenning, Meinig & Partner GbR

(57) **Zusammenfassung**

Die Erfindung bezieht sich auf eine Kathetereinrichtung mit einem Katheter (1), einer Betätigungseinrichtung (8) an einem ersten Ende des Katheters sowie einem mechanischen Transmissionselement (9, 10) zur Übertragung einer Bewegung entlang des Katheters bis zur Betätigungseinrichtung, wobei die Betätigungseinrichtung ein an das Transmissionselement (9, 10) angeschlossenes, von diesem in Bezug auf die Längsrichtung des Katheters in einem ersten Freiheitsgrad antreibbares Koppelelement (14) sowie ein Umsetzelement (15) aufweist, das durch das Koppelelement antreibbar ist und das die Antriebsbewegung wenigstens teilweise in eine Bewegung in einem zweiten Freiheitsgrad umsetzt. Hierdurch ist besonders einfach eine kombinierte Bewegung am distalen Ende des Katheters zur Kompression und Freigabe eines Funktionselements realisierbar.

## Beschreibung

Die Erfindung liegt auf dem Gebiet medizinischer Ausrüstungen und ist insbesondere bei Eingriffen in den menschlichen oder tierischen Körper vorteilhaft einsetzbar.

Im Einzelnen bezieht sich die Erfindung auf eine Kathetereinrichtung, die einen einzuführenden Katheter sowie zusätzliche Elemente aufweist, die eine optimierte Einsetzbarkeit der Kathetereinrichtung gewährleisten.

Ein derartiger Katheter kann durch eine Eintrittsöffnung in ein körpereigenes Gefäß oder einen Leiter, beispielsweise ein Blutgefäß oder einen Harnleiter eingeführt werden, um bestimmte gewünschte Funktionen minimalinvasiv auszuführen. Am Ende des Katheters kann ein Funktionselement vorgesehen sein, das ferngesteuert bestimmte Operationen erlaubt.

Oft besteht das Problem darin, zunächst mittels des Katheters das Funktionselement an die richtige Stelle im Körper zu bringen, ohne auf dem Weg dorthin Körperregionen über das akzeptable Maß hinaus zu schädigen. Zu diesem Zweck kann das Funktionselement während der Einführung entweder abgedeckt oder derart verformt sein, dass die Einführung schädigungsfrei ermöglicht oder zumindest erleichtert ist.

Aus dem Stand der Technik ist eine Anzahl von Kathetereinrichtungen bereits bekannt.

Aus der deutschen Patentschrift DE 103 36 902 B3 ist eine intrakardiale Pumpvorrichtung mit einem Katheter bekannt, der in seinem Endbereich eine Pumpe trägt. Diese kann grundsätzlich einen begrenzt größeren Durchmesser als der Katheter aufweisen, jedoch muss die Pumpe bei zu großem Durchmesser chirurgisch eingeführt und entfernt werden, da sie im Durchmesser grundsätzlich unveränderlich ist.

Aus der deutschen Patentschrift DE 100 59 714 C1 ist eine Pumpe zur Einführung in ein Blutgefäß eines Körpers bekannt, wobei die Pumpe am distalen Ende in eine Kanüle ausläuft, deren Durchmesser veränderlich ist. Beispielsweise befindet sich die Kanüle beim Einführen in das Blutgefäß in einem komprimierten Zustand, der temperaturabhängig in einem Bereich geringerer Temperaturen eingenommen wird. Ist die Kanüle in den Körper eingeschoben, so erwärmt sie sich auf die Körpertemperatur und nimmt dabei eine zweite, expandierte Form aufgrund des Formgedächtnisses ihres Materials ein. Die Kanüle kann zu diesem Zweck beispielsweise aus Nitinol, einem bekannten Material mit Formgedächtniseigenschaften, bestehen. Möglichkeiten, eine derartig aufgeweitete Kanüle wieder in den komprimierten Zustand zu versetzen, um ohne einen chirurgischen Eingriff die Pumpe und den Katheter entnehmen zu können, sind in dem genannten Dokument nicht angesprochen.

Aus der WO 94/05347 ist eine Kathetereinrichtung mit einer Pumpe bekannt, bei der ein Pumpengehäuse und gegebenenfalls auch die Pumpenschaufeln durch relative Längsverschiebung einer Antriebswelle gegenüber einer diese unmittelbar umgebenden Hülse radial aufgestellt werden können.

Vor dem Hintergrund des Standes der Technik stellt sich die Aufgabe, eine Kathetereinrichtung zu schaffen, die mit möglichst einfachen und kostengünstigen konstruktiven Mitteln ein optimiertes und möglichst schädigungsarmes Einführen und Entnehmen des Katheters erlaubt und dabei einen möglichst großen freien Durchgangskanal des Katheters erhält.

Die Aufgabe wird gemäß der Erfindung mit den Merkmalen des Patentanspruchs 1 gelöst.

Dabei sieht die Erfindung eine Kathetereinrichtung vor mit einem Katheter, einer Betätigungseinrichtung an einem ersten Ende des Katheters sowie einem mechanischen Transmissionselement zur Übertragung einer Bewegung entlang des Katheters bis zur Betätigungseinrichtung, wobei die Betätigungseinrichtung ein an das Transmissionselement angeschlossenes, von diesem in Bezug auf die Längsrichtung des Katheters in einem ersten Freiheitsgrad antreibbares Koppelelement sowie ein Umsetzelement aufweist, das durch das Koppelelement antreibbar ist und das die Antriebsbewegung wenigstens teilweise in eine Bewegung in einem zweiten Freiheitsgrad umsetzt.

Der erfindungsgemäße Katheter, der vorzugsweise gänzlich aus biokompatiblen Material / Materialien besteht, kann somit durch eine Öffnung in einen Körper eingeführt, und darauf kann durch das Transmissionselement eine Bewegung zu dem antreibbaren Koppelelement übertragen werden. Eine solche Bewegung kann beispielsweise in Längsrichtung des Katheters in Form von Schieben oder Ziehen erfolgen, oder in Form einer Drehung um die Längsrichtung des Katheters, falls das Transmissionselement die Übertragung einer Drehbewegung erlaubt, beispielsweise wenn das Transmissionselement als ein den Katheter umgebender Schlauch ausgebildet ist.

Die auf das Koppelelement übertragene Bewegung in dem ersten Freiheitsgrad wird von diesem auf ein Umsetzelement übertragen, das die Antriebsbewegung in eine Bewegung in einem zweiten Freiheitsgrad umsetzt. Dabei kann die Bewegung in dem ersten Freiheitsgrad für die Gesamtbewegung des Umsetzelementes und der Betätigungseinrichtung zusätzlich erhalten bleiben.

Beispielsweise kann das Umsetzelement eine Kulissenführung oder eine ähnliche Einrichtung, wie zum Beispiel eine Schnecke zur Umsetzung zwischen einer Bewegung in Längsrichtung des Katheters und einer Drehbewegung um die Längsrichtung aufweisen. In diesem Fall kann beispielsweise eine Zug- oder Schiebebewegüng entlang des Katheters in eine Drehbewegung umgesetzt werden oder umgekehrt.

Vorteilhaft kann die Kulissenführung eine Kulissenbahn und einen Kulissenstein aufweisen, wobei wenigstens eines dieser Elemente, insbesondere der Kulissenstein, in Form eines Führungsstiftes mit dem Katheter fest verbunden ist. Auf diese Weise kann insgesamt eine durch Züge entlang des Katheters aufgebrachte Zug- oder Schiebebewegung eine entsprechende Bewegung des Koppelelementes entlang dem Katheter hervorrufen, die ihrerseits eine Dreh-Schubbewegung des Umsetzelementes erzeugt. Ein Vorteil der Erfindung liegt in diesem Fall darin, dass der Drehanteil der Bewegung nicht entlang des gesamten Katheters übertragen werden muss, sondern erst am Ende des Katheters durch das Umsetzelement erzeugt wird.

Die Dreh-Schubbewegung am Ende des Katheters kann dadurch vorteilhaft verwendet werden, dass sie auf eine Schutzkappe übertragen wird, die ein Funktionselement am Ende des Katheters schützt und mittels der Dreh-Schubbewegung besonders einfach applizierbar ist.

Soll das Transmissionselement speziell eine Bewegung in Längsrichtung des Katheters übertragen, so kann es als Zug, insbesondere nach Art eines Bowdenzuges, oder als eine Gruppe von Zügen ausgebildet sein, die mit dem Koppelelement verbunden sind, entlang des Katheters, insbesondere an dessen Außenseite, verlaufen und nach Einführen des Katheters in ein Gefäß von außerhalb betätigbar sind. Vorteilhaft sind die Züge einzeln an dem Katheter längsverschiebbar geführt, beispielsweise dadurch, dass sie in die Wand des Katheters wenigstens teilweise eingelassen sind.

Es kann auch vorgesehen sein, dass der Zug bzw. die Züge in Führungsvorrichtungen an der Außenseite des Katheters.wie beispielsweise Außenringen geführt sind.

Ist das Transmissionselement als Zug oder Gruppe von Zügen ausgebildet, so sind diese vorteilhaft einzeln an einem gemeinsamen Fixierelement unter Berücksichtigung der durch die gegebene Krümmung des Katheters notwendigen Längenanpassungen festgelegt. Das Fixierelement kann beispielsweise durch das Zusammenwirken von zwei Ringen gebildet sein, zwischen denen die einzelnen Züge festklemmbar sind, nachdem der Katheter eingeführt ist. Danach kann durch Manipulieren des Fixierelementes die Gruppe von Zügen gleichmäßig bewegt werden.

An dem dem Fixierelement gegenüberliegenden Ende des Katheters, an dem das Transmissionselement mit dem Koppelelement verbunden ist, kann dieses als Lagerring ausgebildet sein, mit dem das Umsetzelement drehbar verbunden ist. Dadurch ist sichergestellt, dass in dem Fall, dass das Umsetzelement eine Drehbewegung durchführt, diese nicht auf das Koppelelement und die Züge bzw. allgemein das Transmissionselement übertragen wird. Das Transmissionselement kann somit eine reine Bewegung in Längsrichtung des Katheters ausführen.

Umgekehrt kann auch das Transmissionselement als Schlauch oder rohrförmiges Element ausgebildet und zur Übertragung einer Drehbewegung um die Längsrichtung des Katheters eingerichtet sein. In diesem Fall wird die Drehbewegung auf das Umsetzelement übertragen, das diese in eine Bewegung in Längsrichtung des Katheters umsetzt. Für diesen Fall wird vorteilhaft sichergestellt, dass das Umsetzelement in Längsrichtung des Katheters gegenüber dem Koppelelement verschiebbar ist, beispielsweise dadurch, dass das Koppelelement und das Umsetzelement teleskopartig ineinandergeführt sind.

Die Kathetereinrichtung kann vorteilhaft derart ausgebildet sein, dass eine Schleuse zur Durchführung des Katheters durch eine Öffnung vorgesehen ist, wobei die Schleuse auf der der Betätigungseinrichtung zugewandten Seite eine Form aufweist, die beim Herausziehen des Katheters das Koppelelement zurückhält und dadurch eine Drehbewegung des Umsetzelementes erzeugt.

Wird der Katheter entnommen, d. h. beispielsweise aus einem Blutgefäß herausgezogen, so soll sichergestellt werden, dass das Funktionselement dadurch geschützt wird, dass mittels der Betätigungseinrichtung beispielsweise eine Kappe auf das Funktionselement geschoben wird. Zu diesem Zweck wird mittels der Schleuse sichergestellt, dass auch das Transmissionselement beim Herausziehen des Katheters eine Schubbewegung bewirkt, die an der Betätigungseinrichtung durch die Umsetzeinrichtung in die gewünschte Kombinationsbewegung zum Aufschieben einer Schutzkappe umgesetzt wird.

Es kann dafür gesorgt werden, dass beim weiteren Herausziehen des Katheters die Schleuse mit entfernt wird oder dass nach Betätigung des Transmissionselementes dieses durch die Schleuse hindurchbewegt werden kann.

Eine besonders günstige und vorteilhafte Verwendung der Kathetereinrichtung ist gekennzeichnet durch ein Funktionselement, insbesondere eine Pumpe, am Ende des Katheters sowie eine Schutzkappe, die mit dem Umsetzelement verbunden ist und die in einer ersten Position des Umsetzelementes das Funktionselement aufnimmt und dieses in einer zweiten Position freigibt.

Wenn die Schutzkappe kleinere Innenmaße aufweist als die Außenmaße des Funktionselementes in der freigegebenen Position und das Funktionselement wenigstens teilweise komprimierbar ist, kann durch die erfindungsgemäße Kathetereinrichtung einerseits beim Entnehmen des Funktionselementes dieses durch die Betätigungseinrichtung komprimiert werden und andererseits, wenn die Betätigungseinrichtung ihre zweite Position einnimmt, das Funktionselement freigegeben werden, um zu expandieren. Entsprechende Expansionsbewegungen können beispielsweise durch Verwendung von sogenannten Temperaturgedächtnislegierungen erzielt werden oder dadurch, dass beispielsweise Pumpenschaufeln beim Rotationsbetrieb eines als Pumpe ausgebildeten Funktionselementes selbsttätig in einen Betriebszustand expandieren.

Ein vorteilhaftes Verfahren für ein erfindungsgemäßes Einbringen einer Kathetereinrichtung, die erfindungsgemäß gestaltet ist, sieht vor, dass zunächst der Katheter eingebracht wird, wobei sich die Betätigungseinrichtung in einer ersten Position befindet, und dass darauf mittels des Transmissionselementes das Koppelelement und das Umsetzelement angetrieben werden, um mittels der Betätigungseinrichtung in deren zweiter Position ein Funktionselement freizugeben. Damit ist auf einfache und wenig fehleranfällige Weise sichergestellt, dass ein Katheter mit einem expandierbaren Funktionselement bzw. einer Funktionseinrichtung mit geringem Verletzungsrisiko in ein Gefäß eines Patienten eingebracht und danach in gewünschter Weise expandiert werden kann. Durch die erfindungsgemäße Konstruktion sind auch Funktionsfehler beim Entnehmen der Kathetereinrichtung minimiert.

Im Folgenden wird die Erfindung anhand eines Ausführungsbeispiels in einer Zeichnung anhand mehrerer Figuren gezeigt und anschließend beschrieben. Dabei zeigt
- Fig. 1.: schematisch einen in ein Blutgefäß ein- geführten Katheter, wobei sich das Ende des Katheters im Volumen einer Herzkammer be- findet,
- Fig. 2.: schematisch im Längsschnitt einen Katheter mit einer Betätigungseinrichtung und einem Funktionselement,
- Fig. 3.: im Längsschnitt eine Vorrichtung zur Fixierung von Zügen,
- Fig. 4.: eine alternative Vorrichtung zu der in Fig. 3 gezeigten,
- Fig. 5.: vergrößert im Längsschnitt die Betätigungs- vorrichtung mit einem Teil eines Funktions- elementes sowie
- Fig. 6.: die Betätigungsvorrichtung schematisch in einem Querschnitt.

In dem Ausführungsbeispiel ist speziell die Anwendung der Erfindung auf einen Katheter beschrieben, der an seinem Ende eine Pumpe trägt und in die Blutbahn eines Patienten eingeführt werden kann.

Derartige Pumpen, die beispielsweise in das Herz eines Patienten eingeführt werden und der Blutförderung zur Unterstützung des Herzens dienen, sind grundsätzlich bekannt. Es ist dabei auch bekannt, solche Pumpenstrukturen derart aufzubauen, dass sie nach der Einführung in die Herzkammer im Durchmesser aufgeweitet werden können.

Sinnvoll und im Wesentlichen ein Anwendungsvorteil der vorliegenden Erfindung ist es, dabei einen Mechanismus vorzusehen, der das Funktionselement, das im vorliegenden Fall in der Pumpe besteht, sowohl freigeben kann, so dass es sich potentiell selbsttätig aufweitet, als auch komprimieren kann, um das Funktionselement / die Pumpe mit dem Katheter wieder aus dem Körper entfernen zu können. In der Zwischenzeit soll vorteilhaft die gesamte Vorrichtung im Körper verbleiben und darf in diesem Zustand weder dem Patienten schaden noch den Fluss des Blutes durch den Katheter über ein verträgliches Maß behindern.

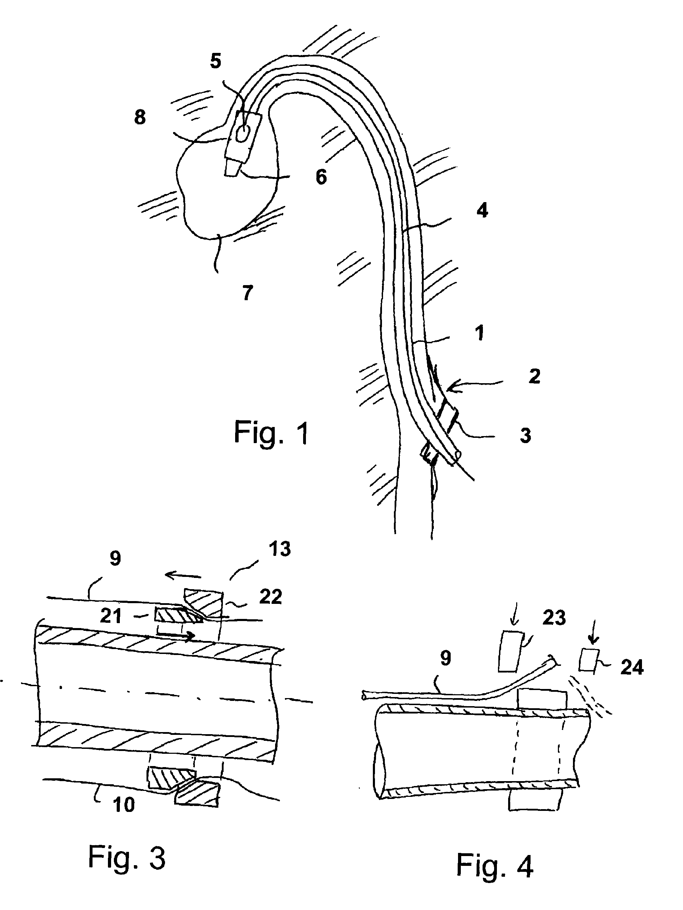

Grundsätzlich wird der Katheter 1, der in der Fig. 1 nur schematisch dargestellt ist, durch eine Eintrittswunde 2 mittels einer Schleuse 3 in das Blutgefäß eingeführt, durch dieses durchgeschoben, bis das Ende 5 des Katheters mit dem Funktionselement 6 in der Herzkammer 7 platziert ist, und in diesem Zustand fixiert, und die Betätigungseinrichtung 8, auf die weiter unten noch genauer eingegangen wird, kann zur Freigabe des Funktionselementes 6 mittels eines Transmissionselementes betätigt werden.

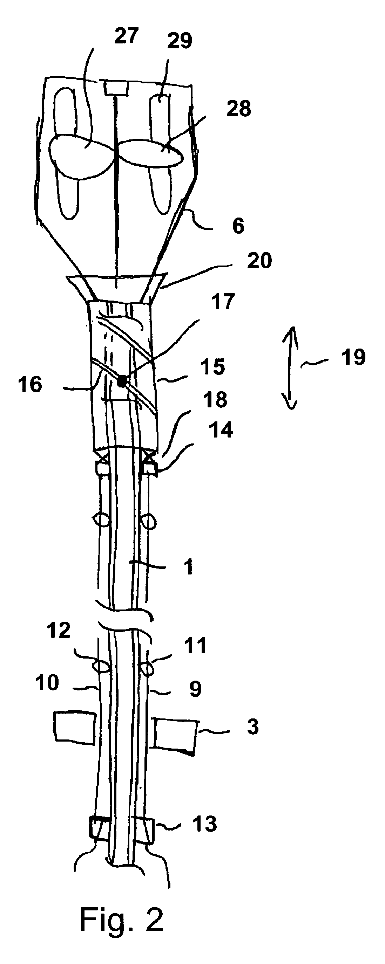

In der Fig. 2 sind das Transmissionselement sowie die Betätigungseinrichtung und das Funktionselement genauer beschrieben. Die Fig. 2 zeigt im Längsschnitt einen Katheter 1, der von einer Gruppe von Zügen 9, 10 umgeben ist, die in Längsrichtung des Katheters 1 an dessen Umfang parallel zueinander verlaufen. Zur Führung der Züge 9, 10 können diese ganz oder teilweise in die Wand des Katheters 1 in Längsrichtung beweglich eingelassen sein oder durch Führungseinrichtungen 11, 12 in Form von Öffnungen oder Schleifen am Umfang des Katheters geführt werden. Wenn die Züge genügend steif und die Führungseinrichtungen 11, 12 genügend eng gesetzt sind, kann außer einer Zugbewegung mittels der Züge auch eine Schubbewegung realisiert werden.

Andererseits ist alternativ denkbar, die Züge durch einen Schlauch zu ersetzen, der den Katheter 1 konzentrisch umgibt und ebenfalls zur Übertragung von Zug- und Schubbewegungen dienen kann. Hierzu ist der Schlauch mit einer entsprechenden axialen Druckfestigkeit auszustatten, ohne dass die Biegesteifigkeit merklich erhöht wird. Entsprechende Technologien sind in der Industrie hinlänglich bekannt und bereits umgesetzt.

Grundsätzlich ist auch die Realisierung des Transmissionselementes durch einen einzigen Zug am Umfang des Katheters denkbar. Bevorzugt ist dabei die Anordnung von Zügen an der Außenseite des Katheters oder wenigstens teilweise in der Wand des Katheters, jedoch ist grundsätzlich auch, insbesondere bei einer geringen Anzahl von Zügen, deren Führung innerhalb des Katheters denkbar.

Die Züge 9, 10 sind gemeinsam am Fixierelement 13 beispielsweise durch Klemmung befestigt. Damit kann durch Manipulation des Fixierelementes 13 die Gesamtheit von Zügen gleichsinnig und in gleichem Maße bewegt werden. Auf das Fixierelement wird weiter unten noch genauer eingegangen.

Zudem ist aus der Fig. 2 ein Koppelelement 14 ersichtlich, das dort als in Längsrichtung des Katheters verschiebbarer Lagerring ausgebildet ist. An diesem Lagerring 14 sind die Züge 9, 10 angesetzt, so dass Zug- oder Druckbewegungen der Züge zunächst auf den Lagerring 14 übertragen werden.

Mit dem Lagerring 14 ist andererseits das Umsetzelement 15 in Form eines mit einer oder mehreren Kulissenbahnen 16 versehenen Zylinders verbunden. Das Koppelelement 14 überträgt Zug- und Schubbewegungen auf den Zylinder 15, in dessen Kulissenbahn ein Führungsstift 17, der einen Kulissenstein bildet, geführt ist. Der Zylinder 15 wird dadurch auf eine schraubenförmige Bewegungsbahn gezwungen, da der Führungsstift 17 in dem Katheter 1 fixiert ist.

Damit das Umsetzelement 15 sich unabhängig von dem Lagerring 14 drehen kann, ist zwischen diesen beiden Elementen eine Drehlagerung 18 beispielsweise in Form eines Gleitlagers vorgesehen, das Schub- und Zugkräfte übertragen kann.

Werden die Züge 9, 10 betätigt, so ergibt sich für den Lagerring 14 ebenso wie für den Zylinder 15 eine Schubbewegung in Richtung des Pfeils 19, die durch die Kulissenführung bei dem Zylinder 15 zusätzlich durch eine Drehbewegung überlagert wird.

In der Fig. 2 ist als Funktionselement 6 am Ende des Katheters 1 eine Blutpumpe dargestellt, die gegenüber dem Durchmesser des Katheters bzw. der Betätigungseinrichtung aufgeweitet ist. Dies ist dadurch möglich, dass das genannte Funktionselement eine expandierte und eine komprimierte Lage einnehmen kann, wobei es in der komprimierten Lage durch eine Schutzkappe 20 am Ende des Betätigungselementes 14, 15 wenigstens teilweise aufgenommen werden kann.

Wird das Umsetzelement 15 zusammen mit der Schutzkappe 20 in einer schraubenartigen Bewegung durch die Züge 9, 10 zurückgezogen, so kann sich die Pumpe 6 im Durchmesser aufweiten, während sie bei einer Schubbewegung der Züge 9, 10 durch die Schraubbewegung der Kappe 20 komprimiert und in der Kappe wenigstens teilweise aufgenommen wird.

Zudem wird durch die Darstellung aus der Fig. 2 deutlich, dass beim Entnehmen des Katheters aus der Eintrittswunde die Schleuse 3 dadurch eine besondere Rolle spielt, dass spätestens bei Anschlagen des Lagerrings 14 an der Schleuse 3 die Schubbewegung der Schutzkappe 20 eingeleitet oder vollendet wird, so dass zumindest beim Passieren der Eintrittswunde das Funktionselement in vollständig komprimierter Form vorliegt.

Die Pumpe ist zum Zweck der Komprimierbarkeit besonders gestaltet, wobei beispielsweise die Rotorblätter der Pumpe einklappbar sind und das Gehäuse eingefaltet werden kann, beispielsweise durch die Herstellung aus einem sogenannten Temperaturgedächtnismaterial, einer Legierung, die bei unterschiedlichen Temperaturen verschiedene Formen annehmen kann. Es ist ebenfalls eine elastisch komprimierbare Konstruktion denkbar, die allein durch Kraftwirkung und/oder durch die Einwirkung des beförderten Fluids in die Wunschposition auf- oder zugeklappt werden kann.

Die Fig. 3 zeigt ein Fixierelement 13 in Form von zwei konischen Ringen 21, 22, zwischen denen die Züge 9, 10 durch axiales Zusammenpressen der Ringe eingeklemmt werden können. Da die Züge einzeln festgelegt werden, können Längenunterschiede, die durch Krümmung des Katheters und entsprechend unterschiedlichen Längenbedarf der radial innen oder außen liegenden Züge entstehen, ausgeglichen werden. Das Fixierelement sollte in diesem Fall nach Einbringen des Katheters die Züge festlegen. Danach kann mittels des Fixierelementes die Gruppe von Zügen gemeinsam betätigt werden.

In Fig. 4 ist eine andere Ausführungsvariante des Fixierelementes dargestellt, bei der die Züge 9, 10 mit einer Quetsch- oder Knickbewegung durch entsprechend bewegliche Elemente 23, 24 festgelegt werden können.

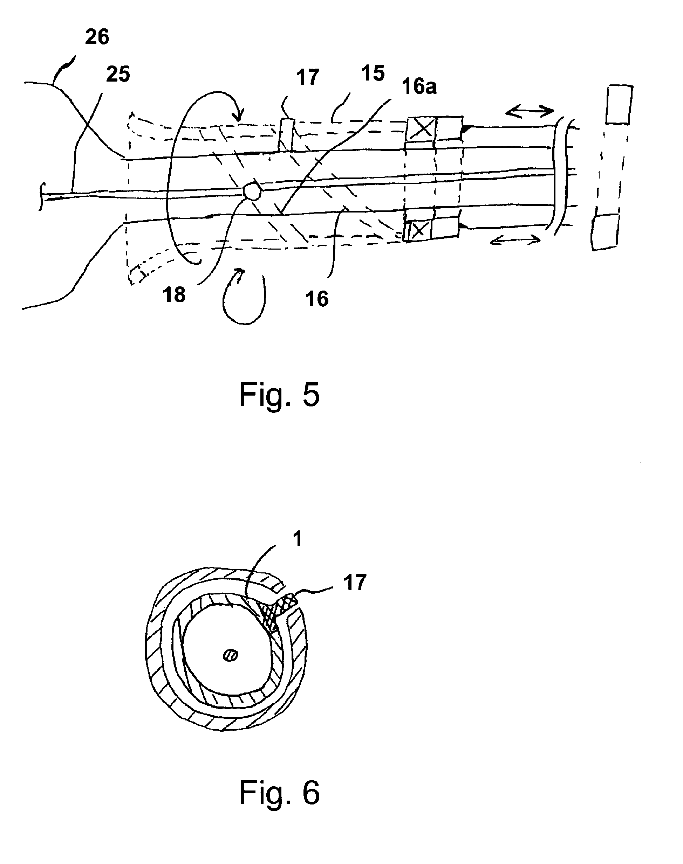

Aus der Fig. 5 geht noch detaillierter als aus Fig. 2 die Funktionsweise des Umsetzelementes 15 hervor, wobei dort mehrere Führungsstifte 17, 17a dargestellt sind, die jeweils an dem Katheter 1 befestigt sind und in verschiedenen parallelen, gegeneinander versetzten schraubenartigen Kulissenbahnen 16, 16a laufen können.

Die Schutzkappe 20 am Ende der Umsetzeinrichtung 15 kann in diese integriert oder mit dieser verbunden sein.

Zudem ist zu erwähnen, dass innerhalb des Katheters 1 eine Welle 25 vorgesehen sein kann, die zum Antrieb der Pumpe 6 dient und in dem Pumpengehäuse 26 gelagert ist. Mittels der Welle 25 werden Rotorblätter 27, 28 angetrieben, und es wird Blut z. B. durch die Öffnungen 29 im Pumpengehäuse 26 befördert. Je nach Auslegung des Rotors/der Pumpe ist auch eine Anströmung des Rotors durch die Öffnungen 29 im Pumpengehäuse 26 und eine axiale Abströmung bzw. eine axiale An- und Abströmung möglich.

In der Fig. 6 ist ein Querschnitt durch den Katheter im.Bereich der Betätigungseinrichtung gezeigt, wobei der in den Katheter 1 integrierte Führungsstift/ Führungsstein 17 dargestellt ist. Der Führungsstift 17 kann in den Katheter entweder eingegossen oder eingeklebt sein und/oder selbst aus dem Material des Katheters 1 bestehen und auch einstückig mit dem Katheter ausgebildet sein. Der Katheter sollte aus einem biegsamen, jedoch festen Kunststoff bestehen, der einen konstanten Querschnitt des Katheters sicherstellt. Wird als Transmissionselement ein Schlauch verwendet, so kann dieser aus demselben Material bestehen wie der Katheter. Werden Züge verwendet, so müssen diese aus einem hochfesten biokompatiblen Stoff wie beispielsweise Edelstahl, Platin oder Silberdraht oder bestimmten Kunststoffen bestehen. Die Züge können mit einer Rasteinrichtung versehen sein, um das Festlegen in unterschiedlichen Längen je nach der vorliegenden Krümmung des Katheters an dem Fixierelement zu erlauben.

Durch die mit der Erfindung erzeugte schraubenartige Bewegung der Betätigungseinrichtung wird das Aufschieben einer Schutzkappe auf das Funktionselement schon im Hinblick auf die günstigeren Reibungsvoraussetzungen wesentlich erleichtert. Durch Aufbringen einer gewindeartigen Oberflächenstruktur auf die Außenseite des Funktionselementes, also insbesondere am Pumpengehäuse und/oder an der Innenseite der Schutzkappe, kann zudem die Kompressionsbewegung erleichtert werden.

Die Erfindung gewährleistet somit eine einfache und zuverlässige Betätigung einer Betätigungseinrichtung zum Schutz eines Funktionselementes während der Positionierung des Katheters in einem sensiblen Bereich innerhalb eines Patientenkörpers.

## Patentansprüche

1. Kathetereinrichtung mit einem Katheter (1), einer Betätigungseinrichtung (8) an einem ersten Ende des Katheters sowie einem mechanischen Transmissionselement (9, 10) zur Übertragung einer Bewegung entlang des Katheters bis zur Betätigungseinrichtung (8), wobei die Betätigungseinrichtung ein an das Transmissionselement angeschlossenes, von diesem in Bezug auf die Längsrichtung des Katheters in einem ersten Freiheitsgrad antreibbares Koppelelement (14) sowie ein Umsetzelement (15) aufweist, das durch das Koppelelement (14) antreibbar ist und das die Antriebsbewegung wenigstens teilweise in eine Bewegung in einem zweiten Freiheitsgrad umsetzt.

2. Kathetereinrichtung nach Anspruch 1, **dadurch gekennzeichnet, dass** das Umsetzelement (15) eine Einrichtung, insbesondere eine Kulissenführung (16, 17) zur Umsetzung zwischen einer Bewegung in Längsrichtung des Katheters und einer Drehbewegung um die Längsrichtung aufweist.

3. Kathetereinrichtung nach Anspruch 2, **dadurch gekennzeichnet, dass** die Kulissenführung (16, 17) eine Kulissenbahn (16) und einen Kulissenstein (17) aufweist, wobei wenigstens eines der Elemente mit dem Katheter (1) fest verbunden ist.

4. Kathetereinrichtung nach Anspruch 1, 2 oder 3, **dadurch gekennzeichnet, dass** das Transmissionselement (9, 10) dazu eingerichtet ist, eine Bewegung in Längsrichtung des Katheters (1) zu übertragen.

5. Kathetereinrichtung nach Anspruch 1, 2, 3 oder 4, **dadurch gekennzeichnet, dass** das Umsetzelement (15) eine Drehbewegung um die Längsrichtung des Katheters im Bereich der Betätigungseinrichtung (8) erzeugt.

6. Kathetereinrichtung nach Anspruch 1 oder einem der folgenden, **dadurch gekennzeichnet, dass** die Verbindung zwischen dem Koppelelement (14) und dem Umsetzelement (15) eine bewegliche Lagerung (18) vorsieht, die eine Relativbewegung des Koppelelementes und des Umsetzelementes in dem zweiten Freiheitsgrad ermöglicht.

7. Kathetereinrichtung nach Anspruch 6, **dadurch gekennzeichnet, dass** das Koppelelement (14) als Lagerring ausgebildet ist, mit dem das Umsetzelement (15) drehbar verbunden ist.

8. Kathetereinrichtung nach Anspruch 1 oder einem der folgenden, **dadurch gekennzeichnet, dass** das Transmissionselement (9, 10) als, insbesondere ungeradzahlige, Anzahl von Zügen ausgebildet ist, die parallel oder helixartig zu dem Katheter (1), insbesondere an dessen Außenseite geführt sind.

9. Kathetereinrichtung nach Anspruch 8, **dadurch gekennzeichnet, dass** die Züge (9, 10) einzeln an dem Katheter in Längsrichtung verschiebbar geführt sind.

10. Kathetereinrichtung nach Anspruch 9, **dadurch gekennzeichnet, dass** die Züge (9, 10) wenigstens teilweise in die Wand des Katheters (1) eingelassen sind.

11. Kathetereinrichtung nach Anspruch 8, 9 oder 10, **dadurch gekennzeichnet, dass** die Züge (9, 10) jeweils einzeln an einem gemeinsamen Fixierelement (13) unter Berücksichtigung der durch die gegebene Krümmung des Katheters notwendigen Längenanpassungen festgelegt sind.

12. Kathetereinrichtung nach einem der Ansprüche 1 bis 7, **dadurch gekennzeichnet, dass** das Transmissionselement als ein den Katheter umgebender Schlauch ausgebildet ist.

13. Kathetereinrichtung nach Anspruch 4 oder einem der folgenden, **dadurch gekennzeichnet, dass** eine Schleuse (3) zur Durchführung des Katheters (1) durch eine Öffnung vorgesehen ist, wobei die Schleuse (3) auf der der Betätigungseinrichtung (8) zugewandten Seite eine Form aufweist, die beim Herausziehen des Katheters das Koppelelement (14) zurückhält und **dadurch** eine Drehbewegung des Umsetzelementes (15) erzeugt.

14. Kathetereinrichtung nach Anspruch 1 oder einem der folgenden, **gekennzeichnet durch** ein Funktionselement (6), insbesondere eine Pumpe, am Ende des Katheters sowie eine Schutzkappe (20), die mit dem Umsetzelement (15) verbunden ist und die in einer ersten Position des Umsetzelementes das Funktionselement (6) aufnimmt und dieses in einer zweiten Position freigibt.

15. Kathetereinrichtung nach Anspruch 14, **dadurch gekennzeichnet, dass** die Schutzkappe (20) kleinere Innenmaße aufweist als die Außenmaße des Funktionselementes in der freigegebenen Position und dass das Funktionselement wenigstens teilweise komprimierbar ist.

16. Verfahren zum Einbringen einer Kathereinrichtung nach einem der vorangehenden Ansprüche, **dadurch gekennzeichnet, dass** zunächst der Katheter eingebracht wird, wobei sich die Betätigungseinrichtung (8) in einer ersten Position befindet, und dass darauf mittels des Transmissionselementes (9, 10) das Koppelelement (14) und das Umsetzelement (15) angetrieben werden, um mittels der Betätigungseinrichtung in deren zweiter Position ein Funktionselement (6) freizugeben.

17. Verfahren nach Anspruch 16, **dadurch gekennzeichnet, dass** vor der Entnahme der Kathetereinrichtung mittels des Transmissionselementes (9, 10) die Betätigungseinrichtung (8) angetrieben wird, um das Funktionselement (6) in eine Schutzkappe (20) einzuführen.

18. Verfahren nach Anspruch 16 oder 17, **dadurch gekennzeichnet, dass** die zum Freigeben des Funktionselementes und die zum Einführen des Funktionselementes in die Schutzkappe erforderlichen Bewegungen der Betätigungseinrichtung gegenläufig sind.
